# FASCICULE DE BREVET EUROPEEN

(11) **EP 3 197 458 B1**
(45) Date de publication et mention de la délivrance du brevet: **31.03.2021**
(21) Numéro de dépôt: 15788101.2
(22) Date de dépôt: 22.09.2015
(51) Int. Cl.: A61K 31/522, A61P 17/00, A61P 11/06, A61P 37/08

(54) **UTILISATION DERMOCOSMETIQUE OU PHARMACEUTIQUE D'UNE COMPOSITION CONTENANT AU MOINS UN INHIBITEUR DE CERTAINES CYTOKINES CHIMIO-ATTRACTANTES**
DERMOKOSMETISCHE ODER PHARMAZEUTISCHE VERWENDUNG EINER ZUSAMMENSETZUNG MIT MINDESTENS EINEM INHIBITOR BESTIMMTER CHEMOATTRAKTANT-ZYTOKINE
DERMOCOSMETIC OR PHARMACEUTICAL USE OF A COMPOSITION CONTAINING AT LEAST ONE INHIBITOR OF CERTAIN CHEMOATTRACTANT CYTOKINES

(30) Priorité: 24.09.2014 FR 1402162
(43) Date de publication de la demande: 02.08.2017
(73) Titulaire: Greenpharma S.A., 45100 Orléans (FR); Centre National de la Recherche Scientifique (C.N.R.S.), 75794 Paris Cedex 16 (FR); Université de Strasbourg, 67000 Strasbourg (FR)
(72) Inventeur: BERNARD, Philippe, F-45240 La Ferté Saint-Aubin (FR); ABBOUD, Dayana, F-67400 Illkirch (FR); GALZI, Jean-Luc, F-67500 Weitbruch (FR); FROSSARD, Nelly, F-67000 Strasbourg (FR); PILLARD, Christelle, F-45100 Orléans (FR); DO, Quoc, Tuan, F-45100 Orléans (FR)
(74) Mandataire: Vial, Lionel
(86) Numéro de dépôt international: PCT/FR2015/000186
(87) Numéro de publication internationale: WO 2016/046457

(56) Documents cités:
- MOHAMMAD NAVID SOLTANI RAD ET AL: "N7-Tosyltheophylline (TsTh): a highly efficient reagent for the one-pot synthesis of N7-alkyltheophyllines from alcohols", SYNTHESIS, vol. 46, no. 10, 25 mars 2014 (2014-03-25) , pages 1380-1388, XP055182266, ISSN: 0039-7881, DOI: 10.1055/s-0033-1341026
- S. MANZINI ET AL: "Pharmacodynamic profile of isbufylline, a new antibronchospastic xanthine devoid of central excitatory actions", ARZNEIMITTEL-FORSCHUNG/DRUG RESEARCH, vol. 40, no. 11, 1990, pages 1205-1213, XP009183680, ISSN: 2194-9379
- VITTORIO DAL PIAZ ET AL: "Phosphodiesterase 4 inhibitors, structurally unrelated to Rolipram, as promising agents for the treatment of asthma and other pathologies", EUROPEAN JOURNAL OF MEDICINAL CHEMISTRY, vol. 35, no. 5, 2000, pages 463-480, XP004330440, ISSN: 0223-5234, DOI: 10.1016/S0223-5234(00)00179-3
- FRANCESCA LEVI-SCHAFFER ET AL: "Diethylene glycol monoethyl ether (Transcutol(R)) displays antiproliferative properties alone and in combination with xanthines", SKIN PHARMACOLOGY, vol. 9, 1996, pages 53-59, XP009183682, ISSN: 1011-0283, DOI: 10.1159/000211390

## Description

La présente description concerne l'utilisation de certains dérivés inhibant les cytokines chimio-attractantes (généralement appelées chimiokines) dans des compositions cosmétiques ou pharmaceutiques. Plus précisément, elle se rapporte à de nouvelles applications desdits dérivés de la théophylline pour traiter des maladies inflammatoires chroniques et les maladies allergiques ; on peut citer à cet égard, les maladies inflammatoires chroniques externes comme les dermatoses (acné, dermatite atopique, psoriasis, eczéma, peau sèche à tendance atopique, rougeurs ...) ou internes comme la maladie de Crohn, l'asthme allergique et la rhinite allergique.

La maladie de Crohn est une maladie inflammatoire chronique de l'intestin. La publication de Grip O. et Janciauskiene S. (Atorvastatin reduces plasma levels of chemokine (CXCL10) in patients with Crohn's disease, PloS One, 2009, 4(5):e5263) montre l'importance des chimiokines dans cette pathologie.

La rhinite allergique est une maladie chronique souvent attachée aux dermatoses chroniques. Un article de Takeuchi S. et al (Changes in thymus- and activation-regulated chemokine (TARC) associated with allergen immunotherapy in patients with perennial allergie rhinitis. J Investig Allergol Clin Immunol, 2005, 15(3), 172-6) montre l'implication des chimiokines dans cette pathologie.

Les dermatoses sont des affections de la peau et des muqueuses, qui se caractérisent par des manifestations inesthétiques comme des rougeurs et des plaques de desquamation. Plusieurs pathologies sont regroupées sous la dénomination de dermatoses. On peut citer, à titre d'exemples non limitatifs, l'eczéma, la dermatite atopique, le psoriasis, les dermites séborrhéiques ou encore l'acné. Ces dermatoses résultent bien souvent de phénomènes inflammatoires et de désordres immunitaires.

Dans le cas de la dermatite atopique, les lésions se caractérisent par une sécheresse cutanée importante et par des lésions inflammatoires : éruptions érythémateuses papuleuses, vésiculeuses, squameuses et très prurigineuses (démangeaisons). Sur le plan histologique, la dermatite atopique se caractérise, comme bien des dermatoses, par une infiltration de lymphocytes, monocytes et de polynucléaires éosinophiles autour des petits vaisseaux et des capillaires. Sur le plan biochimique, il a été montré que les chimiokines sont fortement impliquées dans les dermatoses et dans les maladies inflammatoires chroniques en général. A titre d'exemple, la publication de Saeki et al (Thymus and activation regulated chemokine (TARC)/CCL17 and skin diseases, J Dermatol Sci, 2006, 43(2), 75-84) met en évidence l'importance de la chimiokine CCL17 dans la dermatite atopique. Une autre publication de Vestergaard et al (Overproduction of Th2-specific chemokines in NC/Nga mice exhibiting atopic dermatitis-like lésions, J Clin Invest, 1999, 104(8), 1097-105) met en évidence l'importance de la même chimiokine CCL17 dans les maladies de la peau.

Les chimiokines sont une famille de petites protéines, majoritairement solubles, de 8-14 kD. Leur fonction la plus étudiée est l'attraction et le contrôle de l'état d'activation des cellules du système immunitaire. Certaines chimiokines s'avèrent être de bonnes cibles thérapeutiques pour traiter les maladies inflammatoires chroniques internes et externes chez l'homme ou l'animal.

La présente description met en évidence de nouvelles applications cosmétiques, pharmaceutiques et vétérinaires de certains agents efficaces pour inhiber directement les chimiokines et traiter les maladies inflammatoires chroniques chez l'homme ou l'animal. Dans cette demande de brevet, on utilise le terme générique « pharmaceutique » pour définir aussi bien un produit destiné à une application chez l'homme qu'un produit destiné à une application chez l'animal.

L'invention est telle que définie dans les revendications ci-après. Elle est limitée à une composition cosmétique ou pharmaceutique contenant un composé choisi parmi le 3,7-dihydro-1,3-diméthyl-7-(3-méthyl-2-butényl)-1H-purin-2,6-dione, le 3,7-dihydro-7-butyl-1,3-diméthyl-1H-purine-2,6-dione et le 3,7-dihydro-7-allyl-1,3-diméthyl-1H-purine-2,6-dione pour une utilisation pour traiter les dermatoses, l'acné, la dermatite atopique, le psoriasis, l'eczéma, la sécheresse de peau à tendance atopique et les rougeurs de la peau, ainsi que la maladie de Crohn et la rhinite allergique. La présente description concerne l'utilisation d'une composition dermo-cosmétique ou pharmaceutique destinée à traiter des maladies inflammatoires chroniques de la peau humaine ou animale, caractérisée en ce ladite composition contient, dans un véhicule pharmaceutiquement acceptable, une quantité efficace d'au moins un agent actif répondant à la formule générale (I) : 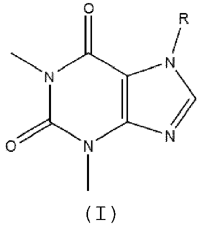 formule dans laquelle :
R représente un groupe (C₁-C₆)alkyle, (C₃-C₆)cycloalkyle, (C₂-C₆)alcényle, groupes dans lesquels un ou plusieurs groupements -CH₂- peuvent être remplacés par -O- ou peuvent être substitués par un ou plusieurs radicaux choisis par les radicaux (C₁-C₆)alkyle, hydroxy et alcoxy, ledit composé de formule (I) pouvant être sous forme d'un sel d'addition basique ou acide.

Les composés de formule (I) peuvent posséder un ou plusieurs centre(s) asymétrique(s) et peuvent alors être isolés sous une forme optiquement active ou sous forme de leur mélange racémique. Les méthodes permettant d'obtenir des formes optiquement actives, par exemple par résolution d'une forme racémique ou par synthèse utilisant des produits de départ racémiques, sont bien connues de l'homme de l'art.

Pour la définition des composés de formule (I), le terme « alkyle » désigne un radical hydrocarboné linéaire ou ramifié ayant avantageusement de 1 à 6 atomes de carbone, tels que méthyle, éthyle, propyle, isopropyle, butyle, isobutyle, tert-butyle, pentyle, néopentyle, n-hexyle. Les groupes alkyle peuvent être substitués par un ou plusieurs groupements hydroxyle et/ou par un ou plusieurs groupements alcoxy tel que défini ci-après.

Le terme « cycloalkyle » désigne un système hydrocarboné cyclique, comprenant de 3 à 6 atomes de carbone et pouvant être mono- ou poly-cyclique. On peut citer notamment les radicaux cyclopropyle et cyclohexyle.

Les groupes « alcoxy » correspondent aux groupes alkyle linéaires ou ramifiés définis ci-dessus reliés par l'intermédiaire d'une liaison -O- (éther). On préfère tout particulièrement les groupes méthoxy, éthoxy, n-propyloxy, *i*-propyloxy, *n*-butoxy, *s*-butoxy, *t*-butoxy, *n*-pentoxy et *s-*pentoxy. Les groupes alcoxy peuvent être substitués par un groupe alkyle comme défini ci-dessus ou par un autre group alcoxy.

Lorsque dans la composition mise en œuvre dans l'utilisation selon la description, l'agent actif est sous la forme d'un sel d'addition acide, ledit acide est avantageusement choisi dans le groupe formé par les acides chlorhydrique, bromhydrique, sulfurique phosphorique, acétique, trifluoacétique, lactique, pyruvique, malonique, succinique, glutarique, fumarique, tartrique, maléique, citrique, ascorbique, méthane- ou éthane-sulfonique et camphorique.

Lorsque, dans la composition mise en œuvre dans l'utilisation selon la description, l'agent actif est sous la forme d'un sel d'addition basique, ladite base est avantageusement choisie dans le groupe formé par l'hydroxyde de sodium ou de potassium, la triéthylamine et la tertiobutylamine.

Parmi les composés de formule (I) utilisables comme agent actif dans les compositions mises en œuvre dans l'utilisation selon la description, on peut notamment citer
3,7-dihydro-1,3-dimethyl-7-(3-methyl-2-butenyl)-1*H*-purin-2,6-dione également appelé prénylthéophylline(codé GPN279) 3,7-dihydro-7-butyl-1,3-dimethyl-1*H*-purine-2,6-dione. 3,7-dihydro-7-isobutyl-1,3-dimethyl-1*H*-purine-2,6-dione. 3,7-dihydro-7-allyl-1,3-dimethyl-1*H*-purine-2,6-dione. 3,7-dihydro-7-(2-ethylhexyl)-1,3-dimethyl-1*H*-purine-2,6-dione

La description concerne notamment une utilisation qui met en œuvre une composition comportant au moins un composé de formule (I) comme agent actif pour traiter des maladies inflammatoires de la peau. Une telle composition peut aussi comporter au moins un agent actif autre que ceux de formule (I) pour exercer au moins une action complémentaire ou synergique.

Selon un autre aspect de la description, la composition mise en œuvre selon la description se présente sous forme de gélules, crème, gel, lotion, lait, émulsion, émulsion biphasique, émulsion H/E ou E/H, pommade, solution, onguent, émulsions triphasiques, huile corporelle, shampoing, nano-capsule, liposome, savon, bâton protecteur des lèvres, bâton et crayon pour maquillage, masques, patchs transdermiques pour applications topiques.

Sous la forme de gel, la composition mise en œuvre selon la description peut comprendre des excipients appropriés, tels que les esters de cellulose, ou d'autres agents gélifiants, tels que le polymère carboxylique « Carbopol® » et la gomme de guar, par exemple.

Sous la forme d'émulsions, les compositions mises en œuvre selon la description jouissent d'une bonne stabilité et peuvent être conservées pendant le temps nécessaire pour l'utilisation à des températures comprises entre 0 et 50 °C, sans qu'il y ait sédimentation des constituants ou séparation des phases.

Selon un autre aspect de la description, dans la composition mise en œuvre selon la description, l' (ou les) agent(s) actif (s) est (sont) mis en place dans des moyens d'encapsulation choisis dans le groupe formé par des microsphères, des liposomes, des glycosphères, des chylomicrons, des macro- micro-, et nanoparticules, des macro-, micro- et nanocapsules.

Selon un autre aspect de la description, dans la composition mise en œuvre selon la description, l' (ou les) agent (s) actif (s) est (sont) absorbé (s) ou adsorbé(s) sur des polymères organiques poudreux, des talcs, de la bentonite ou d'autres supports minéraux en poudre.

Selon un autre aspect de la description, dans la composition mise en œuvre selon la description, l' (ou les) agent(s) actif(s) de formule (I) constitue (nt) de 0,01% à 5% en poids par rapport au poids total de la composition.

Selon un autre aspect de la description, dans la composition mise en œuvre selon la description, l'(ou les) agent (s) actif (s) de formule (I) est (ou sont) sous forme encapsulée et constitue (nt), de préférence de 0,5 à 5% en poids par rapport au poids total de la composition.

La description a, notamment, pour objet l'utilisation d'une composition telle que précédemment définie pour prévenir et/ou traiter des maladies inflammatoires chroniques soit internes, comme la maladie de Crohn, l'asthme allergique et la rhinite allergique, soit externes liées à des activités séborrhéiques, acnéiques, inflammatoires et immunologiques ; une telle composition peut notamment être utilisée pour prévenir et/ou traiter des dermatoses, le psoriasis, la dermatite atopique, ainsi que des états inflammatoires des systèmes cutanés ou pileux induisant des démangeaisons, des irritations, des rougeurs chroniques ou des troubles fonctionnels de la fragilité capillaire.

La description a donc mis en évidence la possibilité d'utiliser les composés de formule (I) comme agent(s) actif(s). Ces composés présentent de très bonnes propriétés pour inhiber certaines chimiokines, comme CCL22 et/ou CCL17.

La description vise donc précisément à proposer une nouvelle utilisation de pharmaceutique, dermatologique ou cosmétique mettant en œuvre des compositions capables de traiter les maladies inflammatoires chroniques internes et externes comme les peaux irritées, lésées ou sensibilisées par des agressions externes ou internes, en général, et des dermatoses, en particulier.

Ainsi, la présente description a pour objet une utilisation cosmétique d'au moins un composé de formule (I) pour inhiber la chimiokine CCL22 et/ou CCL17, pour apaiser les peaux sèches à tendance atopique et/ou pour améliorer et/ou renforcer l'hydratation de la peau. Elle concerne donc des applications pour soigner les pathologies chroniques inflammatoires comme la maladie de Crohn, la rhinite allergique, l'asthme allergique, les dermatoses comme la dermatite atopique, l'eczéma et le psoriasis.

Dans la visée thérapeutique de la description, les dérivés de formule (I) capables d'inhiber la chimiokine CCL22 et/ou CCL17 peuvent être administrés par voie topique mais ils peuvent aussi être administrés par voie orale. Ces dérivés peuvent être utilisés tels quels, sous forme liquide ou en poudre, non purifiés ou purifiés.

Les dérivés de formule (I) capables d'inhiber les chimiokines peuvent, dans les compositions mises en oeuvre, être associés entres eux ou avec d'autres composés venant compléter l'effet voire synergiser cet effet. L'activité protectrice des dérivés de formule (I) vis-à-vis des radicaux libres et des UV est intéressante dans le domaine capillaire, notamment dans le cas d'association avec des substances facilitant le bon état du cuir chevelu et du cheveu. On peut citer les associations avec des mucopolysaccharides, des minéraux, des vitamines, des céramides, des huiles végétales, des substances antiradicalaires, des filtres U.V., des acides de fleurs ou de fruits.

De même, l'activité réparatrice des composés de formule (I) capables d'inhiber les chimiokines est particulièrement intéressante, quand ils sont associés avec des substances ayant un effet cicatrisant comme des protéines, l'acide hyaluronique, des acides aminés, ou avec des substances anti-inflammatoires, anti-âge, après-solaire anti-acné ou anti-dermatose.

Ainsi, les compositions mises en œuvre selon la description sont tout particulièrement adaptées à une application topique pour prévenir et/ou traiter de nombreuses altérations cutanées, notamment comme agent réparateur et/ou protecteur de la peau et du système pileux comme les cheveux, pour lutter contre les agressions externes liées à la pollution, au soleil, au stress oxydatif, au vieillissement et aux pathologies cutanées entrainant un dysfonctionnement de l'homéostasie de l'épiderme ou des cheveux.

Ces compositions cosmétiques mises en œuvre selon la description peuvent aussi prendre la forme de lotions ou solutions dans lesquelles les composés de formule (I) sont sous forme encapsulée, par exemple dans des microsphères. Ces microsphères peuvent, par exemple, être constituées de corps gras, d'agar et d'eau. Les agents actifs peuvent être aussi incorporés dans des vecteurs de type liposomes, glycosphères, dans des chylomicrons, des macro-, micro-, nanoparticules ainsi que les macro-, micro- et nanocapsules et aussi être adsorbés sur des polymères organiques poudreux, les talcs, bentonites et autres supports minéraux.

Pour la préparation des compositions mises en œuvre selon la description, les dérivés de formule (I) peuvent être mélangés aux excipients généralement employés en cosmétique. Les compositions cosmétiques mises en œuvre selon la description peuvent donc contenir des additifs ou des adjuvants usuels en cosmétologie, comme par exemple des agents antibactériens ou des parfums mais aussi des lipides d'extraction et/ou de synthèse, des polymères gélifiants et viscosants, des tensio-actifs, des émulsifiants, des actifs hydro- ou lipo-solubles, des extraits de plantes, des extraits tissulaires, des extraits marins, ou des actifs de synthèse.

L'utilisation dermo-cosmétique ou pharmaceutique des composés de formule (I) comprend tous les produits de soin du corps et de la peau, y compris les produits solaires, protecteurs et bronzants, les produits anti-âges, anti-séborrhéiques, toniques, les produits assurant l'amélioration de l'aspect de la peau y compris le traitement acnéique, le traitement des rougeurs cutanées, le traitement du cuir chevelu et celui de la chute des cheveux.

Les compositions cosmétiques mises en œuvre selon la description peuvent aussi comprendre d'autres agents actifs complémentaires choisis pour leur action, par exemple pour la protection solaire, l'effet anti-rides, l'activité antiradicalaire et antioxydante, l'activité anti-irritante, la nutrition cellulaire, la respiration cellulaire, l'hydratation et la régénération cellulaire, les traitements anti-séborrhéïques, ainsi que d'autres agents actifs ayant une action sur la tonicité cutanée et la protection du cheveu.

Les compositions cosmétiques mises en œuvre selon la description sont, de préférence, à utiliser quotidiennement en les appliquant une ou plusieurs fois par jour.

Les compositions cosmétiques mises en œuvre selon la description sont très bien tolérées, elles ne présentent aucune phototoxicité et leur application sur la peau, pour des périodes de temps prolongées, n'implique aucun effet systémique.

La description concerne aussi l'utilisation de composés de formule (I) pour la préparation de compositions pharmaceutiques présentant une activité anti-inflammatoire et/ou dermoprotectrice. Ces compositions sont utiles pour prévenir et/ou traiter notamment des maladies dermatologiques liées à des activités séborrhéiques, acnéiques, inflammatoires et immunologiques.

En résumé, la description se rapporte donc à l'utilisation d'une composition pharmaceutique, dermatologique ou cosmétique, contenant des composés de formule (I) capables d'inhiber certaines chimiokines, destinée à prévenir et/ou traiter les pathologies et désordres résultant :
- du vieillissement naturel ou prématuré de la peau ou des cheveux ;
- de dermatoses, comme le psoriasis, l'acné et la dermatite atopique ;
- d'états inflammatoires et/ou immunitaires des systèmes cutanés ou pileux, induisant des démangeaisons, irritations, rougeurs chroniques, irritations et démangeaisons persistantes, et des troubles fonctionnels de la fragilité capillaire.

La description est maintenant illustrée par les exemples donnés ci-après.

Les exemples 1 à 5 illustrent des procédés de préparation de dérivés de formule (I) capable d'inhiber la chimiokine CCL17 et/ou CCL22 selon la description.

Les produits de départ sont disponibles dans le commerce ou peuvent être synthétisés par des méthodes classiques connues de l'homme du métier.

### Exemple 1 :

Synthèse du 3,7-dihydro-1,3-dimethyl-7-(3-methyl-2-butenyl)-1*H*-purin-2,6-dione ou prénylthéophylline (codé GPN279)

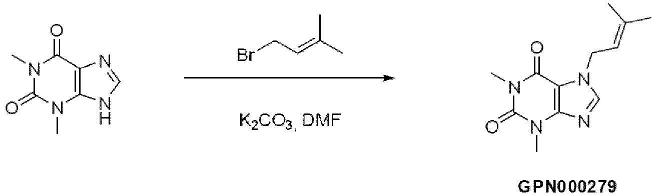

Dans un ballon sous argon, 2 g (0.0111 mol) de théophylline sont dissous dans 30 mL de diméthylformamide puis 2.3 g (1.5 éq.) de carbonate de potassium et 1.54 mL (1.2 éq.) de bromure de prényle sont ajoutés. Le milieu réactionnel est agité 18 heures à 40 °C puis laissé revenir à température ambiante. Il est versé sur de l'eau glacée (environ 40 mL) puis extrait à l'acétate d'éthyle (3 fois 30 mL). Les phases organiques rassemblées sont séchées sur sulfate de magnésium, filtrées et évaporées sous pression réduite. Le brut est purifié par chromatographie sur gel de silice (éther de pétrole / acétate d'éthyle : 5/5, 3/7 puis acétate d'éthyle pur) pour conduire à 2.6 g de GPN279 (rendement : 94%) sous forme d'un solide blanc.

RMN ¹H (250 MHz, CDCl₃) : δ 1.80 (s, 6H, 2 CH₃); 3.42 (s, 3H, NCH₃); 3.59 (s, 3H, NCH₃); 4.92 (d, 2H, *J* = 7.25 Hz, CH₂); 5.44 (m, 1H, CH); 7.54 (s, 1H, CH).

RMN ¹³C (62.5 MHz, CDCl₃: δ 18.3 (CH₃) ; 25.8 (CH₃) ; 28.1 (NCH₃) ; 29.9 (NCH₃) ; 44.7 (CH₂) ; 107.2 (Cq) ; 117.9 (CH) ; 139.8 (Cq) ; 140.3 (CH) ; 148.9 (Cq) ; 151.8 (Cq) ; 155.5 (Cq).

### Exemple 2 :

Synthèse du 3,7-dihydro-7-butyl-1,3-dimethyl-1*H*-purine-2,6-dione (codé GPS008658)

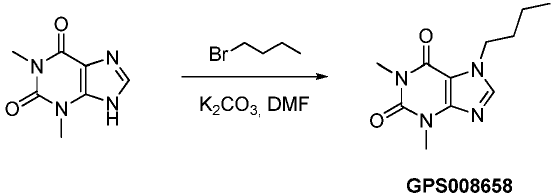

Dans un ballon sous argon, 0.200 g (1.1101 mmol) de théophylline sont dissous dans 3 mL de diméthylformamide puis 0.230 g (1.5 éq.) de carbonate de potassium et 0.14 mL (1.2 éq.) de 1-bromobutane sont ajoutés. Le milieu réactionnel est agité 18 heures à 40 °C puis laissé revenir à température ambiante. Il est versé sur de l'eau glacée (environ 10 mL) puis extrait à l'acétate d'éthyle (3 fois 10 mL). Les phases organiques rassemblées sont lavées avec une solution saturée de chlorure de sodium, sont séchées sur sulfate de magnésium, filtrées et évaporées sous pression réduite. Le brut est purifié par chromatographie sur gel de silice (éther de pétrole / acétate d'éthyle : 5/5 puis 3/7) pour conduire à 0.250 g de GPS008658 (rendement : 95%) sous forme d'un solide blanc.

RMN ¹H (400 MHz, CDCl₃) : δ 0.96 (t, 3H, *J* = 7.2 Hz, CH₃) ; 1.36 (sex, 2H, *J* = 7.2 Hz, CH₂) ; 1.86 (q, 2H, *J* = 7.2 Hz, CH₂) ; 3.42 (s, 3H, NCH₃); 3.60 (s, 3H, NCH₃); 4.29 (t, 2H, *J* = 7.2 Hz, CH₂); 7.53 (s, 1H, CH).

RMN ¹³C (100 MHz, CDCl₃) : δ 13.6 (CH₃) ; 19.7 (CH₂) ; 28.1 (NCH₃) ; 29.9 (NCH₃) ; 33.0 (CH₂) ; 47.2 (CH₂) ; 107.1 (Cq) ; 140.9 (CH) ; 149.0 (Cq) ; 151.8 (Cq) ; 155.3 (Cq).

### Exemple 3 :

Synthèse du 3,7-dihydro-7-isobutyl-1,3-dimethyl-1*H*-purine-2,6-dione (codé GPS008659)

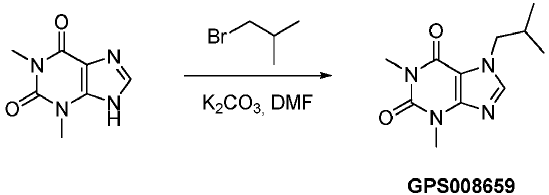

Dans un ballon sous argon, 0.200 g (1.1101 mmol) de théophylline sont dissous dans 3 mL de diméthylformamide puis 0.230 g (1.5 éq.) de carbonate de potassium et 0.14 mL (1.2 éq.) de 1-bromo-2-méthylpropane sont ajoutés. Le milieu réactionnel est agité 18 heures à 40 °C puis laissé revenir à température ambiante. Il est versé sur de l'eau glacée (environ 10 mL) puis extrait à l'acétate d'éthyle (3 fois 10 mL). Les phases organiques rassemblées sont lavées avec une solution saturée de NaCl, séchées sur sulfate de magnésium, filtrées et évaporées sous pression réduite. Le brut est purifié par chromatographie sur gel de silice (éther de pétrole / acétate d'éthyle : 5/5 puis 3/7) pour conduire à 94 mg de GPS008659 (rendement : 35%) sous forme d'un solide blanc.

RMN ¹H (400 MHz, CDCl₃) : δ 0.94 (d, 6H, *J* = 6.4 Hz, 2CH₃);2.23 (sept, 1H, *J* = 6.4 Hz, CH) ; 3.41 (s, 3H, NCH₃); 3.60 (s, 3H, NCH₃); 4.08 (d, 2H, *J* = 7.2 Hz, CH₂) ; 7.50 (s, 1H, CH).

RMN ¹³C (100 MHz, CDCl₃) : δ 19.7 (2CH₃) ; 28.1 (NCH₃) ; 29.8 (CH) ; 29.9 (NCH₃) ; 54.6 (CH₂) ; 107.3 (Cq) ; 141.3 (CH) ; 149.1 (Cq) ; 151.8 (Cq) ; 155.3 (Cq).

### Exemple 4 :

Synthèse du 3,7-dihydro-7-allyl-1,3-dimethyl-1*H*-purine-2,6-dione (codé GPS008660)

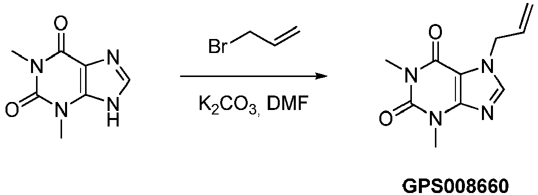

Dans un ballon sous argon, 0.200 g (1.1101 mmol) de théophylline sont dissous dans 3 mL de diméthylformamide puis 0.230 g (1.5 éq.) de carbonate de potassium et 0.12 mL (1.2 éq.) de bromure d'allyle sont ajoutés. Le milieu réactionnel est agité 18 heures à 40 °C puis laissé revenir à température ambiante. Il est versé sur de l'eau glacée (environ 10 mL) puis extrait à l'acétate d'éthyle (3 fois 10 mL). Les phases organiques rassemblées sont lavées avec une solution saturée de NaCl, séchées sur sulfate de magnésium, filtrées et évaporées sous pression réduite. Le brut est purifié par chromatographie sur gel de silice (éther de pétrole / acétate d'éthyle : 5/5 puis 3/7) pour conduire à 230 mg de GPS008660 (rendement : 94%) sous forme d'un solide blanc.

RMN ¹H (400 MHz, CDCl₃) : δ 3.41 (s, 3H, NCH₃) ; 3.60 (s, 3H, NCH₃); 4.95 (d, 2H, *J* = 6.0 Hz, CH₂) ; 5.29 (dd, 2H, *J* = 10.4 et 17.2 Hz, CH₂) ; 6.00 - 6.10 (m, 1H, CH) ; 7.57 (s, 1H, CH).

RMN ¹³C (100 MHz, CDCl₃) : δ 28.1 (NCH₃) ; 29.9 (NCH₃) ; 49.1 (CH₂) ; 107.0 (Cq) ; 119.5 (CH2) ; 132.2 (CH) ; 140.8 (CH) ; 148.9 (Cq) ; 151.8 (Cq) ; 155.3 (Cq) .

### Exemple 5 :

Synthèse du 3,7-dihydro-7-(2-ethylhexyl)-1,3-dimethyl-1*H-*purine-2,6-dione (codé GPS008661)

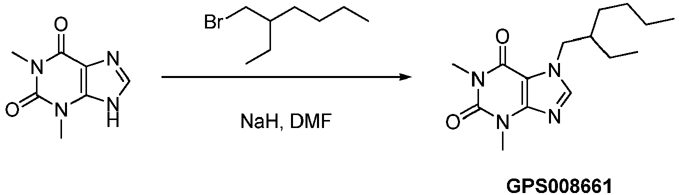

Dans un ballon sous argon, 0.400 g (2.2202 mmol) de théophylline sont dissous dans 10 mL de diméthylformamide puis 98 mg (1.1 éq.) 'hydrure de sodium (60% dans l'huile) sont ajoutés. Le milieu réactionnel est agité 10 minutes à température ambiante puis 0.52 mL(1.3 éq.) de bromure de 2-éthylhexyle sont additionnés. La réaction est agitée 2 jours à température ambiante, versée sur de l'eau glacée (environ 20 mL) puis extraite au dichlorométhane (3 fois 20 mL). Les phases organiques rassemblées sont séchées sur sulfate de magnésium, filtrées et évaporées sous pression réduite. Le brut est purifié par chromatographie sur gel de silice (éther de pétrole / acétate d'éthyle : 5/5) pour conduire à 220 mg de GPS008661 (rendement : 34%) sous forme d'une huile incolore.

RMN ¹H (250 MHz, CDCl₃): δ 0.86-0.94 (m, 6H, 2CH₃); 1.17-1.37 (m, 8H, 4CH₂) ; 1.89-2.02 (m, 1H, CH) ; 3.42 (s, 3H, NCH₃) ; 3.60 (s, 3H, NCH₃) ; 4.16 (d, 2H, *J* = 7.5 Hz, CH₂) ; 7.49 (s, 1H, CH).

RMN ¹³C (100 MHz, CDCl₃) : δ 10.4 (CH₃) ; 14.1 (CH₃) ; 23.1 (CH₂) ; 23.5 (CH₂) ; 28.2 (NCH₃) ; 28.4 (CH₂) ; 29.9 (NCH₃) ; 30.2 (CH₂) ; 40.1 (CH) ; 51.0 (CH₂) ; 107.3 (Cq) ; 141.4 (CH) ; 149.0 (Cq) ; 151.8 (Cq) ; 155.3 (Cq).

L'exemple 6 met en évidence le protocole technique permettant d'enregistrer les effets des produits selon l'invention sur les chimiokines.

### Exemple 6:

Propriétés inhibitrices des dérivés de formule (I) sur les chimiokines.

### Cellules exprimant le récepteur CCR4 des chimiokines

Afin de permettre un enregistrement aisé des réponses associées au récepteur CCR4, le cDNA qui permet son expression est cloné dans le plasmide pIRES (ClonTech) en fusion avec la protéine GFP, selon Vollmer et al. (1999), ce qui permet de mesurer aisément l'expression du récepteur chimérique EGFP-CCR4. Les cellules HEK 293 (ATCC) sont cultivées dans le milieu minimum essentiel MEM (Invitrogen) en présence de 10% de sérum de veau fœtal (Gibco-BRL), 100 U/mL penicilline (Invitrogen), 100 µg/mL streptomycine (Invitrogen) et 2 mM L-glutamine (Invitrogen) à 37°C dans atmosphère saturée en eau et contenant 5% CO2. Les cellules sont transfectées selon la méthode du précipité de phosphate de calcium de Chen et Okayama, 1986, et les cellules ayant incorporé le plasmide d'expression sont sélectionnées à l'aide de 600 µg/mL de geneticine G-418 (PAA) pendant 5 semaines. Les clones sont sélectionnés après avoir été caractérisés par microscopie de fluorescence et analyse cytometrique au FACS (fluorescence-assisted cell sorting).

### Construction de la protéine recombinante Gqi5

Afin de permettre un enregistrement aisé des réponses cellulaires associées au récepteur CCR4, on modifie son couplage naturel à l'inhibition de production d'AMPc vers un couplage à la phospholipase C qui permet des mesures de calcium. On produit dans ce but la protéine chimérique Gqi5 par mutagénèse dirigée des 5 derniers acides aminés de Gq que l'on remplace par leurs homologues de Gi. Les cellules exprimant le récepteur CCR4 sont transfectées par le plasmide pCNA3.1 dans lequel le cDNA codant Gqi5 a été incorporé. Les enregistrements de réponses calciques sont réalisés le lendemain de la transfection.

### Enregistrement des réponses calciques cellulaires et de leur inhibition par les molécules de la collection GPN :

Les cellules sont chargées en indicateur fluorescent du calcium, INDO-1, selon le protocole fourni par le fournisseur (Molecular probes), distribuées dans des puits de microplaques et placées dans un lecteur-pipetteur de fluorescence (FlexStation, Molecular devices). L'élévation de calcium intracellulaire est mesurée par changement d'intensité de fluorescence à 401 et 475 nm (excitation 355 nm). Des points de mesures sont enregistrés chaques 5s pendant 150s pour chaque puits. Les molécules sont testées en présence de 5 nM de chimiokine CCL17 (commerciale)

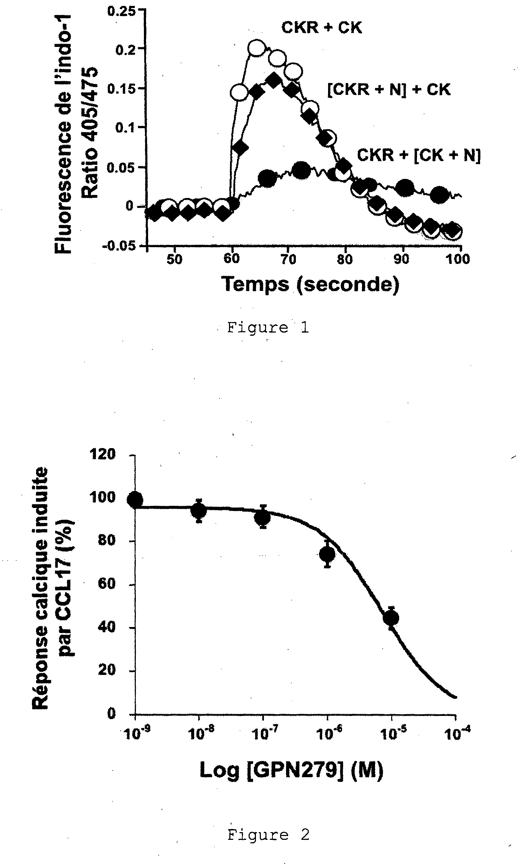

La figure 1 illustre de manière générale les résultats obtenus : l'addition de chimiokine (CK) à des cellules exprimant le récepteur de chimiokine (CKR) déclenche une libération de calcium intracellulaire recelée par le changement de fluorescence de la sonde calcique Indo-1. La réponse se développe en quelques secondes et s'atténue avec le temps pour atteindre le retour en 2 minutes environ pour une concentration de 5 à 10 nM de la chimiokine CCL17 sur des cellules HEK 293 exprimant CCR4. Si l'on préincube les molécules à tester avec la chimiokine [CK+N] et qu'on ajoute le mélange aux cellules, on observe une forte diminution de l'amplitude de la réponse si la molécule à tester est un neutraligand de la chimiokine. Inversement lorsqu'on préincube le neutraligand avec les cellules exprimant le récepteur [CKR + N] on observe une bien moindre diminution de l'amplitude de la réponse.

Ce protocole a été employé pour identifier les neutraligands de CCL17 présentés dans le tableau 1.

*L'affinité apparente des molécules de GPN279* est déterminée par l'établissement d'une relation dose-effet de l'inhibition de la réponse calcique ainsi que présenté en figure 2 et les résultats obtenus sont reportés dans le tableau 1.

### Tableau 1 : relations structure/fonction des dérivés de formule (I)

**TABLEAU 1:**

| **Cpd** | **Référence** | **Structure** | **Inhibition des réponses calciques induites par CCL17 (%)** | **Inhibition des réponses calciques induites par CCL22 (%)** |
|---|---|---|---|---|
| 1 | GPN279 | | 42±4 | 0 |
| 2 | Theophylline | | 0 | 0 |
| 3 | Caféine | | 0 | 0 |
| 4 | GPN062 | | 0 | 0 |
| 5 | IBMX | | 0 | 0 |
| 6 | GPS008658 | | 40±9 | 32±8 |
| 7 | GPS008659 | | 36±7 | 5±5 |
| 8 | G PS008660 | | 19±5 | 0 |
| 9 | G PS008661 | | 0 | 0 |

Les molécules sont testées à la concentration de 10 µM. La concentration de CCL17 est de 5 nM.

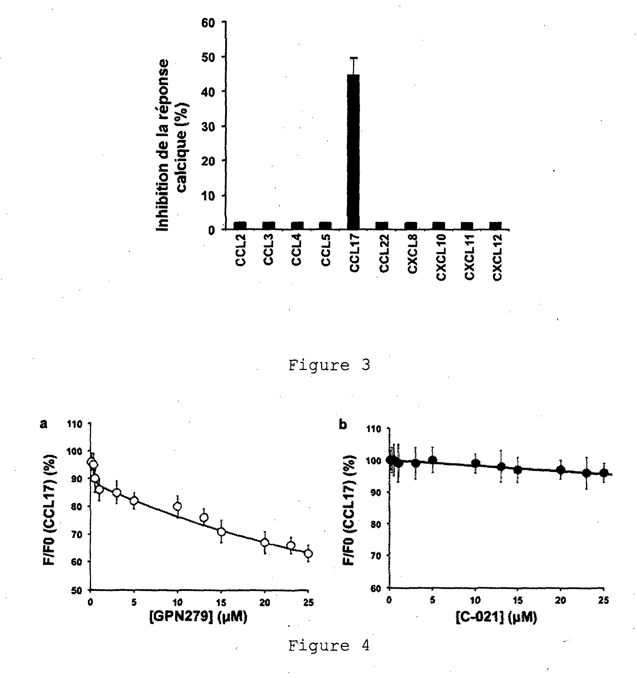

*L'analyse de la sélectivité des molécules* est réalisée par mesure d'inhibition (à la concentration de 10 µM) de la réponse évoquée par d'autres chimiokines (5 nM) sur leurs récepteurs respectifs. Ainsi, dans la figure 3, les molécules ont été testée sur les paires CCL2/CCR2, CCL3-5/CCR5, CCL17/CCR4, CCL22/CCR4, CXCL8/CXCR8, CXCL10/CXCR2, CXCL11/CXCR3, CXCL12/CXCR4

La figure 3 donne la sélectivité du neutraligand de CCL17 (GPN279) sur les chimiokines indiquées et leurs récepteurs respectifs.

### Détermination de l'affinité d'interaction du neutraligand de CCL17 par extinction de fluorescence des résidus de tryptophane.

Cette mesure est réalisée comme décrit dans Hachet-Haas et al. (Small neutralizing molecules to inhibit actions of the chemokine CXCL12, J Biol Chem, 2008, 283(34), 23189-99). Brièvement : les mesures de fluorescence sont réalisées sur un spectrofluorimètre Fluorolog 3 (JobinYvon/Spex) dans une cuvette en quartz. CCL17 (1,5 µM (tampon HEPES sans albumine) dans 1 mL volume final) est excitée à 285 nm and son spectre d'émission est enregistré entre 300 et 400 nm après chaque ajout d'un aliquot de molécule à tester (1 pL). Les mesures sont effectuées à 20°C et les solutions sont agitées à l'aide d'un barreau magnétique. Les spectres d'émission enregistré »s sont corrigés par soustraction du spectre d'émission du composés à tester seul.

La figure 4 indique qu'une extinction de fluorescence de CCL17 est observée uniquement avec le neutraligand (GPN 279) et non pas avec l'antagoniste du récepteur CCR4 (molécule C-021). L'allure de la courbe de titration de CCL17 (1,5 µM) par GPN 279 est biphasique, indiquant la présence vraisemblable de plusieurs sites de liaison pour GPN 279, dont un au moins est de haute affinité. L'ajustement des traces par l'équation (RL)²+(RL) × (-Ro-Lo-KD)+RoxLo=0 avec (RL) = ((Ro+Lo+KD) ± ((-Ro-Lo-KD)² -4×Ro×Lo)^{1/2})/2 permet d'estimer l'affinité pour chaque site : KD< 50 nM pour le site de haute affinité et KD= 57 µM pour le/les sites de basse affinité.

Il n'y a pas de signal pour la molécule C-021.

La figure 4a montre l'inhibition de la fluorescence intrinsèque de CCL17 par le neutraligand GPN 279 (cercles blancs). La figure 4b montre que l'antagoniste du récepteur CCR4, C-021, ne présente pas une diminution de fluorescence du tryptophane (cercles noirs).

L'exemple 6bis permet de montrer comment sont sélectionnés les candidats actifs non cytotoxiques.

### Exemple 6bis:

### Propriétés des dérivés sur la migration de kératinocytes et cytotoxicité

L'activité de la molécule GPN 279 a été évaluée dans un essai de migration de kératinocytes humains réalisé selon un test de cicatrisation d'un tapis cellulaire. Il est effectivement connu que dans les pathologies chroniques une migration importante des cellules peut favoriser la formation de prurit, une des manifestations de la dermatose. Les kératinocytes humains immortalisés obtenus auprès du DKFZ (Heidelberg, Allemagne) sont cultivés dans du milieu de Dulbecco modifié par Eagle (DMEM, InVitrogen) complémenté par 10% de sérum de veau fœtal (Gibco-BRL) et 100 U/ml de pénicilline (Invitrogen), 100 µg/ml de streptomycine (Invitrogen) et 2 mM L-glutamine (Invitrogen) à 37°C et 5% CO2. Les puits d'une plaque de culture 24 puits sont ensemencés avec 200.000 cellules et la culture est maintenue jusqu'à formation d'un tapis confluent. Le tapis est alors endommagé en effectuant un grattage linéaire à l'aide pointe de pipette stérile de 0.1-10µL. Le milieu est alors remplacé pour éliminer les cellules et leurs débris et la culture est poursuivie dans différentes conditions expérimentales : (i) sans aucun ajout, (ii) ajout de 100 ng/ml de CCL17 et (iii) ajout d'un mélange contenant 100 ng/ml de CCL17 et la molécule à tester, par exemple GPN 279. L'effet des molécules seules est aussi enregistré dans des puits différents. Afin de bloquer la prolifération cellulaire, chaque puits reçoit une dose de 1µg/ml de mytomycine (Sigma-Aldrich). Les plaques de culture sont disposées dans l'appareil IncuCyte (Essen Bioscience) qui enregistre la croissance du tapis cellulaire par imagerie transmise en conditions de culture cellulaire (37°C, 5%CO2, atmosphère saturée d'eau) pendant 72h. Des images en contraste de phase sont acquises chaque 2 heures. La cicatrisation est définie (quantifiée) comme le rapport de largeur de la cicatrice au temps t par rapport au temps 0. L'expérience est répétée trois fois par condition.

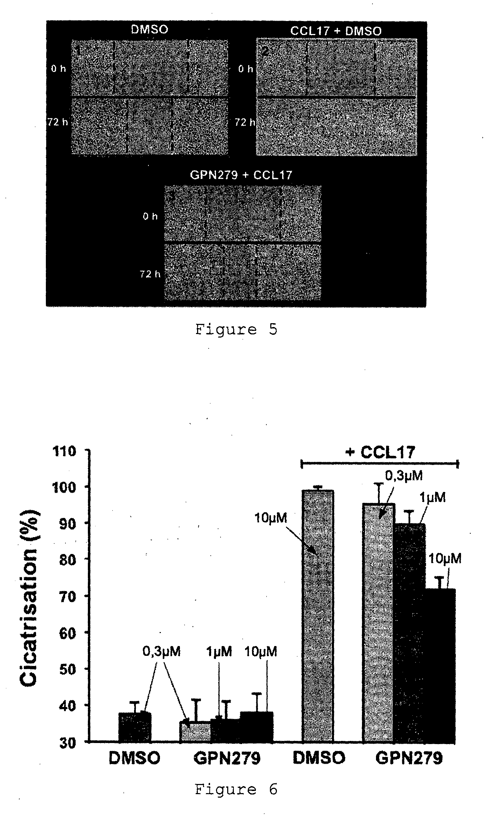

Les résultats obtenus sont rapportés dans la figure 5

La figure 5 montre la migration de kératinocytes humains sous l'effet de GPN279

La figure 5 montre des photographies des tapis de cellules Hacat humaine après blessure au temps 0 et après 72h de culture en présence d'excipient seul (DMSO, panneau 1), de CCL17 et d'excipient (CCL17 + DMSO, panneau 2) et de CCL17 et GPN 279 (CCL17 + GPN279, panneau 3)

La figure indique que la migration cellulaire est fortement accélérée par la chimiokine CCL17 qui agit sur les récepteurs CCR4 présents dans les cellules Hacat. Cet effet de la chimiokine CCL17 est aboli par la molécule GPN279.

La figure 6 montre la relation effet-dose de GPN279 sur la cicatrisation du tapis de cellules HaCat , la molécule GPN279 n'a pas d'effet lorsqu'elle est appliquée seule et son effet est dépendant de la dose (0.3 µM colonne rose; 1 µM colonne violette ; 10 µM colonne bleue).

### Mesure de cytotoxicité

Le test d'activité métabolique est un test colorimétrique (WST-1 assay - Ozyme) reposant sur la mesure de l'activité de l'enzyme mitochondriale succinate-tetrazolium reductase clivant le sel de tétrazolium WST-1 ((4-[3-(4-Iodophenyl)-2-(4-nitrophenyl)-2H-5-tetrazolio]-1,3 benzene disulfonate)) en un dérivé hydrosoluble, le formazan qui est un colorant absorbant la lumière dans la fenêtre 420-480 nm. Les cellules HaCat sont distribuées à raison de 200.000 cellules par puits dans une plaque de culture à 24 puits, dans un volume final de 500 pL. Elles sont cultivées pendant 24h avant d'ajouter de la mitomycine C (1 µg/ml) et le composé à tester. La plaque de culture est incubée pendant 72h à 37°C, 5%CO2, en atmosphère saturée d'eau. Le milieu est ensuite remplacé par du milieu frais complémenté de réactif WST-1 puis incubé pendant 2 heures à 37°C. Les plaques sont agitées pour homogénéiser la couleur dans les puits et l'absorbance est mesurée à 450 nm dans un lecteur de plaques multipuits. Les mesures sont réalisées en triplicats. L'activité enzymatique requérant l'intégrité cellulaire, l'absorbance sera d'autant plus forte que l'état métabolique des cellules est bon.

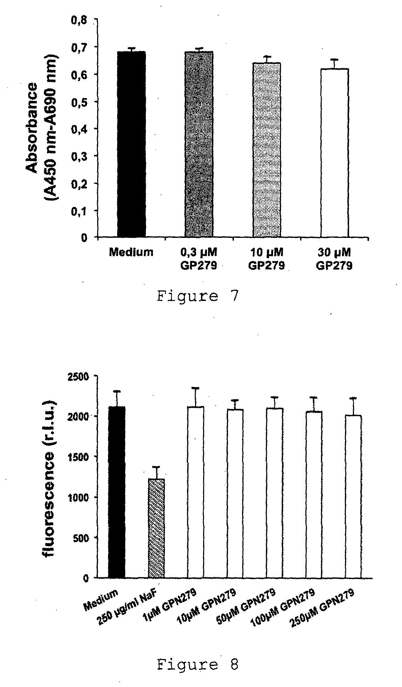

Activité cytotoxique *in vitro:* les cultures de cellules HaCat sont traitées avec différentes concentrations de molécule:0,3µM (barres gris sombre), 10 µM (barres gris clair) ou 30 µM (barres blanches) de la molécule GPN279. L'activité de l'enzyme formazan-reductase mitochondiale est enregistrée à l'aide du substrat chromogène WST-1 qui se transforme en produit coloré, le formazan, détecté à 450 nm. Comme le montre la figure 7, la molécule GPN 279 n'affecte pas l'activité enzymatique.

**Exemple 7:** cytotoxicité de GPN279 sur des monocytes humains Les monocytes sont ensemencés dans des plaques 96 puits (approx 5000 cellules/puit de 200 µl) pour le test à l'alamar blue. Les cellules sont incubées avec du LPS (lipopolysaccharide) et avec GPN279 à 5 concentrations.

Après 24h, la fluorescence de l'alamar blue est dosée.

La figure 8 montre la mesure de la cytotoxicité par l'Alamar Blue et indique que GPN279 n'est pas cytotoxique.

### Exemple 8: absence d'inhibition des cyclooxygénases par GPN279

La ressemblance de GPN279 avec des composés inhibant les cyclooxygenases suggère que le mécanisme possible pourrait passer par leur inhibition. Nous montrons ici que ce n'est pas le cas.

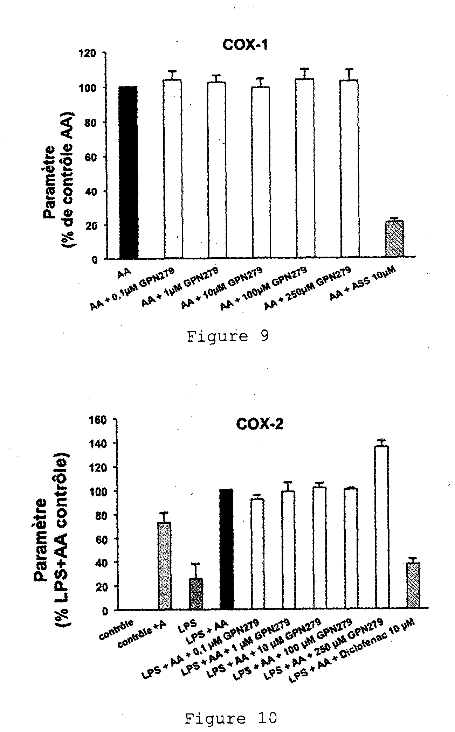

Détermination de l'activité des cyclooxygénases COX 1 et COX 2 sur des monocytes humains en culture.
COX-1 : la libération de prostaglandine E2 (PGE2) médiée par COX-1 est déterminée par la méthode de l'acide arachidonique. Les monocytes sont traités avec l'acide arachidonique (AA) en présence de GPN279 à 5 concentrations qui sont ajoutées 15 min avant l'acide arachidonique. Après 15 minutes de stimulation, l'activité de COX-1 est mesurée par détermination de la production de PGE2. L'aspirine (ASS) un inhibiteur connu de COX-1 est utilisée comme inhibiteur contrôle. La figure 9 indique que GPN279 n'est pas un inhibiteur de COX-1 à une concentration allant jusqu'à 250 uM.
COX-2 : les monocytes sont incubés avec le LPS (10 U/ml) pendant 24h afin d'induire la synthèse de COX-2. Après remplacement du milieu de culture (sans sérum), les cellules sont traitées avec GPN279 à 5 concentrations et le diclofenac (10 µM) est utilisé comme inhibiteur contrôle de cox-2. L'acide arachidonique est ajouté 15 minutes après les composés à tester, et 15 minutes plus tard, la production de PGE2 est déterminée.

La figure 10 montre que GPN279 est sans effet jusqu'à la dose de 100 µM, et induit une légère augmentation d'activité de COX-2 au-delà.

### Exemple 9:

Cet exemple présente une formulation de la composition selon l'invention.

| **NOM COMMERCIAL** | **NOM INCI** | **% DANS LA FORMULE** |
|---|---|---|
| Simulsol 165 | PEG-100 Stearate/ Glyceryl Stearate | 3 |
| Montanov L | C14-22 Alcohol/ C12-20 Alkyl Glucoside | 2 |
| Huile de carthame | Carthamus Tinctorius Seed Oil | 2 |
| Beurre de karité | Butyrospermum Parkii Butter | 4 |
| Céramidone | Octyldodecyl PCA | 3 |
| Phytosqualane | Squalane | 5 |
| DUB MCT 5545 | Caprylic/ Capric Triglycérides | 7 |
| Cire d'abeille | Cera Alba | 1 |
| Acide sorbique | Sorbic acid | 0.1 |
| Glycérine | Glycerin | 2 |
| Sorbitol | Sorbitol | 3 |
| Aristoflex AVC | Ammonium Acryloyldimethyltaurate/ VP copolymer | 1.3 |
| Gomme xanthane | Xanthan gum | 0.2 |
| Nicotinamide | Niacinamide | 1 |
| Lipacide C8G | Capryloyl glycine | 0.5 |
| Eau purifié | Aqua | Qsp pour 100 |
| GPN279 | - | 3 |

On applique cette composition tous les jours pendant 1 mois sur la peau du visage d'un homme de 20 ans souffrant de dermatite atopique sur l'ensemble du visage; cette application est faite à raison d'environ 2 g/jour pour le visage complet. On constate la disparition progressive totale des zones d'inflammation à la fin du traitement.

On a proposé ci-après des ingrédients pouvant être associés avec les compositions pour application topique afin d'amplifier l'effet ou atteindre des synergies d'action, notamment avec la formulation du présent exemple 6 (les quantités sont données en pourcentage en poids) :
a) des ingrédients ayant des propriétés anti-inflammatoires couramment utilisés dans les produits cosmétiques notamment le nicotinamide (1 à 4%), l'acide 18 béta glycyrrhétinique (0.01 à 1%), l'alpha bisabolol (0.1 à 1%) ;
b) la glycine ayant un effet apaisant (de 1 à 3%) ;
c) les micro-particules d'argent (0.1%) qui permettent de réguler, stabiliser la flore microbienne et éviter la surinfection ;
d) un agent anti-adhésion bactérienne pour limiter l'adhésion et la prolifération de Staphylococcus aureus comme le Téflose® de Solabia composé de propanediol, rhamnose, glucose et acide glucuronique (2 à 4%) ;
e) les insaponifiables de beurre de karité et de tournesol pour renforcer la barrière cutanée et améliorer l'hydratation de la peau. (0.5 à 2%).
f) un extrait de chicorée de 1 à 3% (comme la Védérine® de Silab) pour le renforcement et la récupération de la barrière cutanée ;
g) un dérivé d'huile de pépins de framboise et de succinate de vitamine E (Raspberry seed oil/ Tocopheryl succinate/ Aminopropanediol esters) à 1% comme le Vitaskin® E de Solabia ;
h) l'octyldodécyl PCA dosé de 0.5 à 5% pour stimuler la synthèse des lipides épidermiques ;
i) une huile végétale de carthame ou onagre stabilisée sous une forme céramide-like comme l' »Omega 6 Ceramide® Safflower » et l' »Omega 6 Ceramide® Evening primrose » de Solabia pour améliorer la cohésion cellulaire (0.1 à1%) ;
j) un mélange optimisé d'huile d'argan, de beurre de karité et de cire d'orge, comme dans le « Stimutex® AS » de DSM, pour une action anti-inflammatoire et pour diminuer la libération d'histamine (2 à 5%);
k) un mélange d'extrait d'écorce de bouleau blanc et de *Scrophularia nodosa,* comme dans le Protectol® de Greentech, pour ses propriétés anti-inflammatoires du fait de la présence d'acide bétulinique (1 et 3%);
l) un mélange de lactose et de protéine de lait à 0.5% comme dans le Modukine™ de CLR pour ses propriétés anti-inflammatoires.

## Revendications

1. Composition cosmétique ou pharmaceutique pour une utilisation pour traiter des maladies inflammatoires chroniques externes ou internes chez l'homme ou l'animal, contenant, dans un véhicule acceptable, une quantité efficace d'au moins un agent actif constitué par un composé répondant à la formule générale (I) 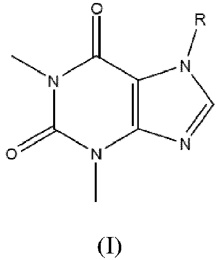 ledit composé étant choisi dans le groupe formé par le 3,7-dihydro-1,3-diméthyl-7-(3-méthyl-2-butényl)-1*H*-purin-2,6-dione, le 3,7-dihydro-7-butyl-1,3-diméthyl-1*H*-purine-2,6-dione, ou le 3,7-dihydro-7-allyl-1,3-diméthyl-1*H*-purine-2,6-dione ;
ledit composé de formule (I) pouvant être sous la forme de sel d'addition,
pour traiter des maladies inflammatoires chroniques, soit externes, sélectionnées dans le groupe constitué des dermatoses, de l'acné, de la dermatite atopique, du psoriasis, de l'eczéma, de la sécheresse de peau à tendance atopique et des rougeurs de la peau, soit internes, sélectionnées dans le groupe constitué de la maladie de Crohn, et de la rhinite allergique.

2. Composition pour une utilisation selon la revendication 1, **caractérisée en ce que** son agent actif est un sel d'addition acide, ledit acide étant choisi dans le groupe formé par les acides chlorhydrique, bromhydrique, sulfurique, phosphorique, acétique, trifluoacétique, lactique, pyruvique, malonique, succinique, glutarique, fumarique, tartrique, maléique, citrique, ascorbique, méthane- ou éthane-sulfonique et camphorique.

3. Composition pour une utilisation selon la revendication 1, **caractérisée en ce que** son agent actif est un sel d'addition basique, ladite base étant choisie dans le groupe formé par l'hydroxyde de sodium ou de potassium, la triéthylamine et la tertiobutylamine.

4. Composition pour une utilisation selon l'une des revendications 1 à 3, **caractérisée en ce qu'**elle contient de 0,01 à 5% en poids d'agent(s) actif(s) de formule (I) par rapport au poids total de la composition.

5. Composition pour une utilisation selon l'une des revendications 1 à 4, **caractérisée en ce que** son (ou ses) agent (s) actif (s) de formule (I) est (sont) sous forme de poudre, ou est (sont) absorbé(s) sur des polymères organiques poudreux, notamment talcs ou bentonites, ou est (sont) sous forme encapsulée.

6. Composition pour une utilisation selon la revendication 5, **caractérisée en ce que**, lorsque son (ses) agent(s) actif(s) de formule (I) est (sont) sous forme encapsulée, le (ou les) moyen(s) d'encapsulation est (ou sont) choisi(s) dans le groupe formé par des microsphères, des liposomes, des glycosphères, des chylomicrons, des macro-, micro-, et nano-particules, des macro-, micro- et nano-capsules.

7. Composition pour une utilisation selon l'une des revendications 1 à 6, destinée à une utilisation cosmétique, **caractérisée en ce qu'**elle contient au moins un agent actif complémentaire autre qu'un composé de formule (I), ledit agent complémentaire étant pris dans le groupe formé par les agents de protection solaire, les agents antirides à activité antiradicalaire, antioxydante, anti-irritante, les agents favorisant la nutrition cellulaire, la respiration cellulaire, l'hydratation cellulaire, la régénération cellulaire, les traitements anti-séborrhéiques, la tonicité cutanée, la protection des cheveux, les agents cicatrisants, l'acide hyaluronique, les acides aminés, les agents anti-âge et les agents après-soleil.

8. Composition pour une utilisation selon l'une des revendications 1 à 7, destinée à une utilisation cosmétique, **caractérisée en ce qu'**elle est formulée sous forme de lotion, gel, émulsion, crème ou lait, émulsion tri-phasique, huile corporelle, shampoing, masque, onguent, pommade, bâton et crayon pour maquillage, nano-capsules, liposomes ou patch transdermique pour applications topiques.

9. Composition pour une utilisation selon l'une des revendications 1 à 8, **caractérisée en ce qu'**elle contient au moins un produit pris dans le groupe formé par des mucopolysaccharides, des vitamines, des céramides, des huiles végétales et des agents efficaces dans les dermatoses.

10. Composition pour une utilisation selon l'une des revendications 1 à 9, destinée à une utilisation cosmétique, **caractérisée en ce qu'**elle contient au moins un produit pris dans le groupe formé par des agents antibactériens, des parfums, des lipides d'extraction et/ou de synthèse, des polymères gélifiants et viscosants, des tensio-actifs, des émulsifiants, des extraits de plantes, des extraits tissulaires, des extraits marins, des actifs hydro- ou lipo-solubles, des actifs de synthèse.

11. Composition pour une utilisation selon l'une des revendications 1 à 10, **caractérisée en ce qu'**elle est administrable par voie topique ou orale.

12. Composition pour une utilisation selon l'une des revendications 1 à 11, pour traiter, sur un sujet animal ou humain, des maladies dermatologiques liées à des activités séborrhéiques, acnéiques et immunologiques.

## Patentansprüche

1. Kosmetische oder pharmazeutische Zubereitung zum Gebrauch in der Behandlung chronischer externer oder interner Entzündungskrankheiten bei Mensch oder Tier, in einem verträglichen Vehikel eine wirksame Menge mindestens eines Wirkstoffes enthaltend, der eine Verbindung der allgemeinen Formel (I) ist 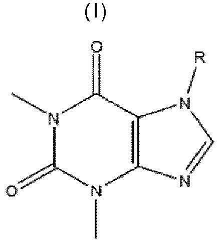 wobei die genannte Verbindung aus der Gruppe gewählt wird, bestehend aus 3,7-Dihydro-1,3-dimethyl-7-(3-methyl-2-butenyl)-1*H*-purin-2,6-dion, 3,7-Dihydro-7-butyl-1,3-dimethyl-1*H*-purin-2,6-dion und 3,7-Dihydro-7-allyl-1,3-dimethyl-1*H*-purin-2,6-dion, wobei die genannte Verbindung der Formel (I) in Form eines Additionssalzes vorliegen kann,
zur Behandlung chronischer Entzündungskrankheiten, entweder externer, gewählt aus der Gruppe, bestehend aus Dermatosen, Akne, atopische Dermatitis, Psoriasis, Ekzem, Hauttrockenheit mit atopischer Tendenz und Hautrötungen, oder interner, gewählt aus der Gruppe, bestehend aus Crohn-Krankheit und allergischer Rhinitis.

2. Zubereitung zum Gebrauch nach Patentanspruch 1, **dadurch gekennzeichnet, dass** der Wirkstoff ein Säureadditionssalz ist, wobei die genannte Säure aus der Gruppe gewählt wird, bestehend aus Salzsäure, Bromsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Zitronensäure, Ascorbinsäure, Methan- oder Ethansulphonsäure und Kampfersäure.

3. Zubereitung zum Gebrauch nach Patentanspruch 1, **dadurch gekennzeichnet, dass** der Wirkstoff ein Basenadditionssalz ist, wobei die genannte Base aus der Gruppe gewählt wird, bestehend aus Natrium- oder Kaliumhydroxid, Triethylamin und tert-Butylamin.

4. Zubereitung zum Gebrauch nach einem der Patentansprüche 1 bis 3, **dadurch gekennzeichnet, dass** sie 0,01 bis 5 Gewichtsprozent Wirkstoff/e der Formel (I) bezogen auf das Gesamtgewicht der Zubereitung enthält.

5. Zubereitung zum Gebrauch nach einem der Patentansprüche 1 bis 4, **dadurch gekennzeichnet, dass** ihr/e Wirkstoff/e der Formel (I) als Pulver vorliegt/vorliegen oder an pulverförmige organische Polymere absorbiert ist/sind, insbesondere an Talke oder Bentonite, oder verkapselt vorliegt/vorliegen.

6. Zubereitung zum Gebrauch nach Patentanspruch 5, **dadurch gekennzeichnet, dass** in dem Fall, dass ihr/e Wirkstoff/e der Formel (I) verkapselt vorliegt/vorliegen, das/die Verkapselungsmittel aus der Gruppe gewählt wird/werden, bestehend aus Mikrosphären, Liposomen, Glycosphären, Chylomikronen, Makro-, Mikro- und Nanopartikeln, Makro-, Mikro- und Nanokapseln.

7. Zubereitung zum Gebrauch nach einem der Patentansprüche 1 bis 6, zu kosmetischem Gebrauch bestimmt, **dadurch gekennzeichnet, dass** sie mindestens einen zusätzlichen Wirkstoff, der nicht der Formel (I) entspricht, wobei der genannte zusätzliche Wirkstoff aus der Gruppe gewählt wird, bestehend aus Sonnenschutzmitteln, Antifaltenmitteln mit Schutz gegen freie Radikale, Antioxydationsmitteln, Antireizmitteln, Mitteln zur Förderung der Zellernährung, der Zellatmung, der Zellenhydrierung, der Zellregeneration, Mitteln für antiseborrhoische Therapie, zur Hautspannung, zum Schutz der Haare, Vernarbungsmitteln, Hyaluronsäure, Aminosäuren, Antialterungsmitteln und "After-Sun"-Mitteln.

8. Zubereitung zum Gebrauch nach einem der Patentansprüche 1 bis 7, zu kosmetischem Gebrauch bestimmt, **dadurch gekennzeichnet, dass** sie als Lotion, Gel, Emulsion, Creme oder Milch, dreiphasige Emulsion, Körperpflegeöl, Shampoo, Maske, Salbe, Pomade, Kosmetikstift, Nanokapseln, Liposomen oder transdermischer Patch zur örtlichen Anwendung formuliert ist.

9. Zubereitung zum Gebrauch nach einem der Patentansprüche 1 bis 8, **dadurch gekennzeichnet, dass** sie mindestens ein Produkt enthält, gewählt aus der Gruppe, bestehend aus Mucopolysacchariden, Vitaminen, Ceramiden, Pflanzenölen und Wirkstoffen gegen Dermatosen.

10. Zubereitung zum Gebrauch nach einem der Patentansprüche 1 bis 9, zu kosmetischem Gebrauch bestimmt, **dadurch gekennzeichnet, dass** sie mindestens ein Produkt enthält, gewählt aus der Gruppe, bestehend aus antibakteriellen Mitteln, Parfums, extrahierten und/oder synthetisierten Ölen, gelbildenden und Viskosität erhöhenden Polymeren, Tensiden, Emulgatoren, Pflanzenextrakten, Gewebsextrakten, Meeresextrakten, wasser- oder fettlöslichen Wirkstoffen, Sythesewirkstoffen.

11. Zubereitung zum Gebrauch nach einem der Patentansprüche 1 bis 10, **dadurch gekennzeichnet, dass** sie örtlich oder oral verabreicht werden kann.

12. Zubereitung zum Gebrauch nach einem der Patentansprüche 1 bis 11 zur Behandlung der Hautkrankheiten, die mit seborrhoischer, akneartiger und immunologischer Aktivität verbunden sind, an einem Tier oder menschlichen Patienten.

## Claims

1. Cosmetic or pharmaceutical composition for use for treating chronic external or internal inflammatory diseases in human or animal, containing, in an acceptable vehicle, an effective amount of at least one active agent consisting of a compound of the general formula (I) 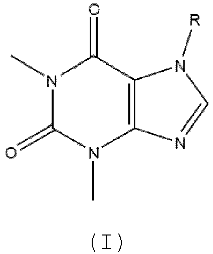 said compound being selected from the group formed by 3,7-dihydro-1,3-dimethyl-7-(3-methyl-2-butenyl-1H-purin-2,6-dione, 3,7-dihydro-7-butyl-1,3-dimethyl-1H-purine-2,6-dione, or 3,7-dihydro-7-allyl-1,3-dimethyl-1H-purine-2,6-dione;
said compound of formula (I) possibly being in the form of an addition salt,
to treat chronic inflammatory diseases, either external, selected from the group consisting of dermatoses, acne, atopic dermatitis, psoriasis, eczema, dryness of atopic-prone skin and redness of the skin, or internal, selected from the group consisting of Crohn's disease and allergic rhinitis.

2. Composition for use according to claim 1, **characterized in that** its active agent is an acid addition salt, said acid being selected from the group formed by hydrochloric, hydrobromic, sulfuric, phosphoric, acetic, trifluoacetic, lactic, pyruvic, malonic, succinic, glutaric, fumaric, tartaric, maleic, citric, ascorbic, methane- or ethane-sulfonic and camphoric acids.

3. Composition for use according to claim 1, **characterized in that** its active agent is a basic addition salt, said base being selected from the group formed by sodium or potassium hydroxide, triethylamine and tert-butylamine.

4. Composition for use according to one of claims 1 to 3, **characterized in that** it contains from 0.01 to 5% by weight of active agent(s) of formula (I) relative to the total weight of the composition.

5. Composition for use according to one of claims 1 to 4, **characterized in that** its active agent(s) of formula (I) is (are) in powder form, or is (are) absorbed on powdery organic polymers, in particular talcs or bentonites, or is (are) in encapsulated form.

6. Composition for use according to claim 5, **characterized in that**, when its active agent (s) of formula (I) is (are) in encapsulated form, the means of encapsulation is (or are) selected from the group formed by microspheres, liposomes, glycospheres, chylomicrons, macro-, micro-, and nano-particles, macro-, micro- and nano-capsules.

7. Composition for use according to one of claims 1 to 6, intended for a cosmetic use, **characterized in that** it contains at least one additional active agent other than a compound of formula (I), said additional agent being taken from the group formed by sun protection agents, anti-wrinkle agents with anti-free radical, antioxidant, anti-irritant activity, agents promoting cell nutrition, cell respiration, cell hydration, cell regeneration, anti-seborrheic treatments, skin tone, hair protection, healing agents, hyaluronic acid, amino acids, anti-aging agents and after-sun agents.

8. Composition for use according to one of claims 1 to 7, intended for a cosmetic use, **characterized in that** it is formulated in the form of a lotion, gel, emulsion, cream or milk, three-phase emulsion, body oil, shampoo, mask, ointment, balm, stick and pencil for makeup, nano-capsules, liposomes or transdermal patch for topical applications.

9. Composition for use according to one of claims 1 to 8, **characterized in that** it contains at least one product taken from the group formed by mucopolysaccharides, vitamins, ceramides, vegetable oils and agents effective in dermatoses.

10. Composition for use according to one of claims 1 to 9, intended for a cosmetic use, **characterized in that** it contains at least one product taken from the group formed by antibacterial agents, perfumes, extraction and/or synthetic lipids, gelling and viscous polymers, surfactants, emulsifiers, plant extracts, tissue extracts, marine extracts, water-soluble or lipo-soluble active ingredients, synthetic active ingredients.

11. Composition for use according to one of claims 1 to 10, **characterized in that** it is administrable topically or orally.

12. Composition for use according to one of claims 1 to 11, for treating, on an animal or human subject, dermatological diseases associated to seborrheic, acne and immunological activities.
